# EUROPEAN PATENT APPLICATION

(11) **EP 2 305 123 A1**
(43) Date of publication of application: **06.04.2011**
(21) Application number: 10182227.8
(22) Date of filing: 29.09.2010
(51) Int. Cl.: A61B 8/00, G01S 7/52, G01S 15/89

(54) **Ultrasonic diagnostic apparatus and method for operating the same**

(30) Priority: 30.09.2009 JP 2009227546
(71) Applicant: Fujifilm Corporation, Minato-ku Tokyo (JP)
(72) Inventor: Sato, Tomoo, Kanagawa (JP)
(74) Representative: Klunker . Schmitt-Nilson . Hirsch

(57) **Abstract**

An ultrasonic diagnostic apparatus (10) includes an ultrasonic probe (11) and a processing device (12). The ultrasonic probe includes an array transducer (21) having plural ultrasonic transducers (31) for transmitting ultrasonic beams to an inside of a subject and receiving echo waves to output echo signals, and a first scan line data generator (26) for focusing the echo signals received by each channel of the array transducer and generating two or more first scan line data after a transmission of the ultrasonic beams. A data set of the first scan line data is sent to the processing device. The processing device includes a second scan line data generator (44) for synthesizing second scan line data from the two or more first scan line data concerning a same scan line and obtained from the transmissions in different directions, and an image generator (46) for generating an ultrasonic image of the subject based on the second scan line data.

## Description

### FIELD OF THE INVENTION

The present invention relates to an ultrasonic diagnostic apparatus for producing ultrasonic tomographic images to perform diagnosis, and a method for operating the same.

### BACKGROUND OF THE INVENTION

An ultrasonic diagnostic apparatus enables realtime observation of a cross-sectional image or ultrasonic tomographic image of an object of interest with small stress on a patient. Because of this, the ultrasonic diagnostic apparatus is used in abdomen examinations, mammary examinations, thyroid examinations, and the like. The ultrasonic diagnostic apparatus is composed of an ultrasonic probe and a processing device (main body). Being contacted with the body of the patient, the ultrasonic probe transmits ultrasonic beams or waves to an object of interest inside of the body of the patient and receives echo therefrom, and then inputs echo signals to the processing device. Thereafter, the processing device produces a cross-sectional image of the patient based on the echo signals to display it on a monitor.

Conventionally, a so-called line-by-line method is known as a method to obtain echo signals on one to four scan lines per single transmission/reception of ultrasonic beams while the transmitted ultrasonic beams narrow down to a small size. The line-by-line method has a disadvantage that the frame rate reduces as the size of the ultrasonic beams reduces to improve image quality. Recently, a so-called zone sonography method is known which uses wide ultrasonic beams to simultaneously obtain larger number of echo signals on scan lines to make the image quality improvement compatible with a high frame rate (see Japanese Patent Laid-Open Publication No. 2003-180688 corresponding to U.S. Patent No. 6,251,073).

A common ultrasonic diagnostic apparatus is provided with an ultrasonic probe having ultrasonic transducers aligned in a row for transmitting and receiving ultrasonic waves. Using the row of the ultrasonic transducers, a cross-sectional image of the patient is generated and the generated image is displayed. Recently, a technique in which ultrasonic transducers are arranged in two dimensions to obtain echo signals in two dimensions is known. With this technique, an image showing the inside the body of a patient is generated and displayed in three dimensions (See PCT publication No. 02/1729 corresponding to Japanese Patent Publication of translated version 2004-506497 and U.S. Patent No. 6,375,617)

Normally, the ultrasonic probe and the processing device are connected via a cable which transmits and receives echo signals and the like. In this case, a cable may interfere with operation during diagnosis, which impairs operability of the ultrasonic probe. Accordingly, it is desired to reduce a diameter of cable with less number of wires or to use wireless connection.

Typically, when the echo signal is converted into a digital signal, one frame of an echo signal has approximately 40 MB. To transfer the echo signals at 20 frames/ second, a transfer rate as high as approximately 1 GB/ second is required. This makes difficult to reduce the number of wires to reduce the diameter of the cable between the probe and the processing device, and extremely difficult to make the connection wireless. Even if the wireless transfer at 1 GB/ second becomes possible, it cannot be commercially available in consideration of power and frequency bands occupied for the data transfer.

PCT publication No. 02/17297 discloses a technique to transmit the echo signal to the processing device after a partial analog delay-and-sum is performed in one of two directions in the ultrasonic probe. In this case, the analog signals are transmitted between the ultrasonic probe and the processing device. When the signal intensity becomes weak, a signal-to-noise (SN) ratio deteriorates. Simply performing the delay-and-sum before the transmission of echo signals to the processing device does not reduce the amount of data transfer between the probe and the processing device enough to reduce the diameter of the cable, let alone wireless connection.

### SUMMARY OF THE INVENTION

An object of the present invention is to provide an ultrasonic diagnostic apparatus for reducing an amount of data transferred from an ultrasonic probe to a processing device and a method for operating the same.

In order to achieve the above and other objects, the ultrasonic diagnostic apparatus of the present invention includes an ultrasonic probe and a processing device. The ultrasonic probe includes an array transducer and a first scan line data generator. The array transducer has plural ultrasonic transducers for transmitting ultrasonic beams to an inside of a subject and receiving echo waves to output echo signals. The first scan line data generator focuses the echo signals received by each channel of the array transducer and generates two or more first scan line data after a transmission of the ultrasonic beams. A data set of the first scan line data is sent to the processing device. The processing device includes a second scan line data generator and an image generator. The second scan line data generator synthesizes second scan line data from the two or more first scan line data concerning a same scan line, which are obtained from the transmissions in different directions. The image generator generates an ultrasonic image of the subject based on the second scan line data.

It is preferable that the number of lines of ultrasonic beams is less than the number of the scan lines, and a wide beam is transmitted such that the adjacent ultrasonic beams are partly overlapped.

It is preferable that the first scan line data generator generates the two or more first scan line data concerning the same scan line using the echo signals obtained with successive ultrasonic beams, and the second scan line data is generated from the two or more first scan line data concerning the same scan line.

It is preferable that the ultrasonic probe further includes a controller for controlling a width to which the ultrasonic beams converge, and the first scan line data generator changes the number of the first scan line data depending on the width to which the ultrasonic beams converge.

It is preferable that the controller changes a shift amount of the ultrasonic beams in accordance with the width to which the ultrasonic beams converge.

It is preferable that the ultrasonic probe further includes a conversion section for performing quadrature detection to the echo signal to convert the echo signal into a complex baseband signal, and the first scan line data generator and the second scan line data generator generate the first scan line data and the second scan line data, respectively, based on the complex baseband signal.

It is preferable that the first scan line data is sent from the ultrasonic probe to the processing device via wireless communication.

The method for operating an ultrasonic diagnostic apparatus having an ultrasonic probe and a processing device of the present invention includes a transmission step, an outputting step, a focusing step, a sending step, a synthesizing step, and an image generation step. In the transmission step, plural ultrasonic transducers of an array transducer provided in the ultrasonic probe are driven to transmit ultrasonic beams to an inside of a subject. In the outputting step, echo waves are received with the plural ultrasonic transducers and echo signals are outputted from the ultrasonic transducers the after a transmission of the ultrasonic beams. In the focusing step, the echo signals received by each channel of the array transducer are focused, and two or more first scan line data are generated in a first scan line data generator in the ultrasonic probe. In the sending step, a data set of the first scan line data is sent from the ultrasonic probe to the processing device. In the synthesizing step, in a second scan line data generator in the processing device, second scan line data is synthesized from the two or more first scan line data concerning the same scan line and obtained from the transmissions in different directions. In the image generation step, an ultrasonic image of the subject is generated based on the second scan line data in an image generator in the processing device.

According to the present invention, the amount of data transferred from the ultrasonic probe to the processing device is reduced, to be more specific, to an extent that the wireless connection between the ultrasonic probe and the processing device is possible.

### BRIEF DESCRIPTION OF THE DRAWINGS

The above and other objects and advantages of the present invention will be more apparent from the following detailed description of the preferred embodiments when read in connection with the accompanied drawings, wherein:
Figure 1 is a block diagram showing a configuration of an ultrasonic probe;
Figure 2 is a block diagram of a processing device of the ultrasonic diagnostic apparatus;
Figure 3 is an explanatory view of scan lines;
Figures 4A to 4C are explanatory views showing transmission of ultrasonic beams and generation of first scan line data;
Figure 5 is an explanatory view of the first scan line data transferred from the ultrasonic probe to the processing device;
Figures 6A to 6D are explanatory views showing generation of second scan line data from the first scan line data; and
Figure 7 is an explanatory view showing generation of the second scan line data of all the scan lines by second reception focusing processes.

### DESCRIPTION OF THE PREFERRED EMBODIMENTS

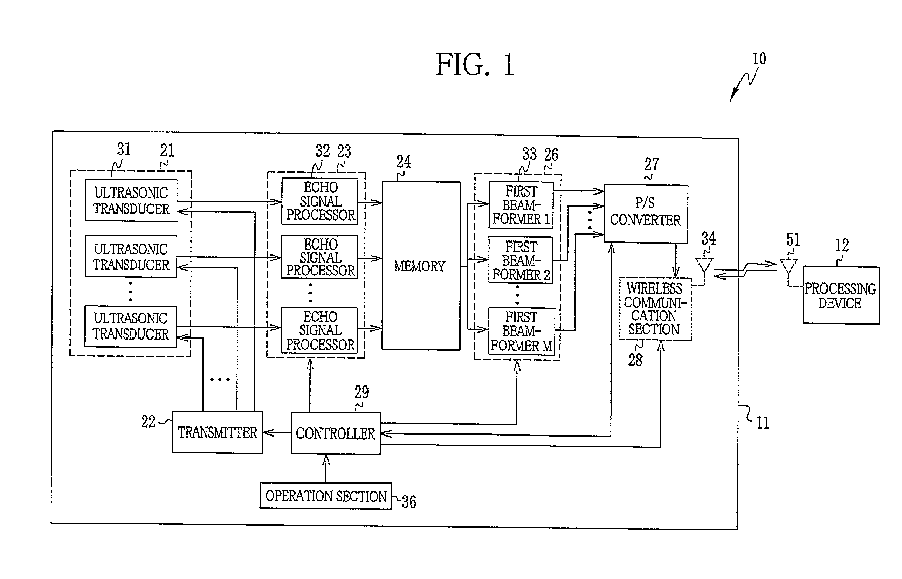

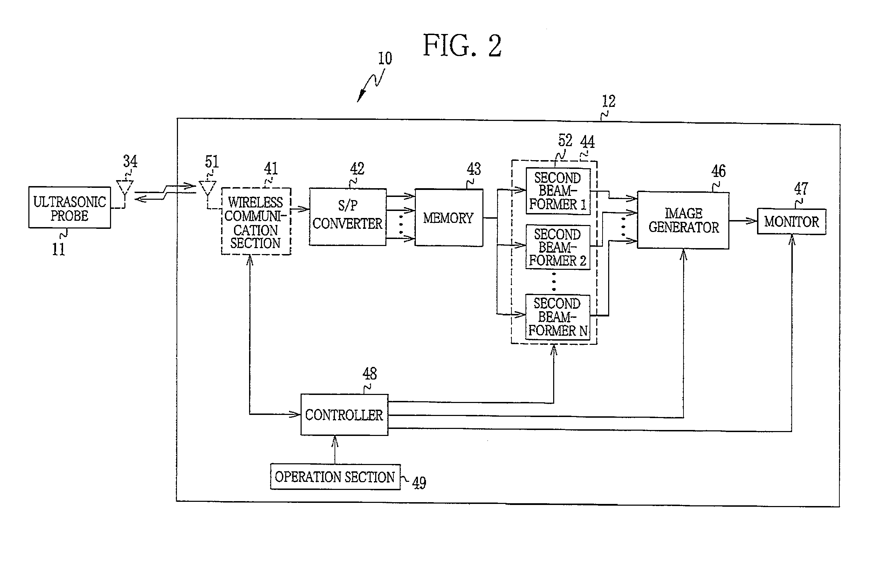

As shown in Figs. 1, an ultrasonic diagnostic apparatus 10 is composed of an ultrasonic probe 11 and a processing device 12 or main body. The ultrasonic probe 11 is contacted to a subject or patient when in use. The ultrasonic probe 11 transmits ultrasonic beams or waves to an object of interest inside the body of the patient, and receives echo therefrom. The ultrasonic probe 11 generates first scan line data based on the received echo and then wirelessly transmits the first scan line data to the processing device 12. The processing device 12 generates second scan line data from the first scan line data received from the ultrasonic probe 11, and generates a cross-sectional image or ultrasonic tomographic image of the object of interest. The generated cross-sectional image is displayed on a monitor 47 (see Fig. 2).

The ultrasonic probe 11 is composed of an ultrasonic array transducer (array transducer) 21, a transmitter 22, a receiver 23, a memory 24, a first scan line data generator 26, a parallel/serial (P/S) converter 27, a wireless communication section 28, and a controller 29.

The array transducer 21 is composed of plural ultrasonic transducers 31 aligned in a row. Each ultrasonic transducer 31 is provided at around a tip of the ultrasonic probe 11. The ultrasonic transducers 31 transmit ultrasonic beams or waves to the inside of the body of the patient and receive echo therefrom. For example, the array transducer 21 is composed of 200 ultrasonic transducers 31, and performs transmission/reception with 200 channels. Each of the ultrasonic transducers 31 is actuated by a drive signal inputted from the transmitter 22. To transmit ultrasonic beams from the array transducer 21, K (K is an integer equal to or larger than 2) adjacent ultrasonic transducers 31, for example, 12 adjacent ultrasonic transducers are selectively driven as a group while each of the selected ultrasonic transducers 31 is driven with a delay. Thereby, the ultrasonic beams transmitted by the K ultrasonic transducers 31 are narrowed or focused to a predetermined size at predetermined position and depth inside the body of a patient. The L ultrasonic transducers 31, for example, 100 ultrasonic transducers 31 receive the echo reflected from the inside of the body, and then output analog echo signals (RF signals).

The transmitter 22 generates a drive signal and inputs the generated drive signal to each of the ultrasonic transducers 31. The drive signal causes the ultrasonic transducer 31 to pulse-oscillate to transmit ultrasonic beams or waves. The transmitter 22 selects the K ultrasonic transducers 31 to transmit ultrasonic waves and sets their delay timing to adjust or change a direction or position to oscillate or transmit the ultrasonic beams, focal depth, and the size of the focal region. The transmitter 22 sequentially drives a group of the K ultrasonic transducers 31 from one of the ends to the other end of the array transducer 21 to transmit the ultrasonic beams or waves while shifting or changing the group of the K ultrasonic transducers to be driven at a constant pitch or interval, for example, by 4 channels. In this case, 8 channels are overlapped between the adjacent groups.

Immediately after the transmission of ultrasonic beams, the receiver 23 obtains analog echo signals from the ultrasonic transducers 31 which received the echo waves, and converts them into digital echo signals. The receiver 23 is provided with echo signal processors 32 with the number equal to the number of the ultrasonic transducers 31 such that the echo signal processor 32 is in one-to-one correspondence with the ultrasonic transducer 31. When an analog echo signal is inputted from the corresponding ultrasonic transducer 31, the echo signal processor 32 amplifies the analog echo signal and performs quadrature detection to convert the analog echo signal into a complex baseband signal. The complex baseband signal is then A/D converted into a digital echo signal. The digital echo signal is stored in the memory 24. For each transmission of ultrasonic beams, the receiver 23 reads echo signals from 100 channels selected corresponding to the transmission position of ultrasonic waves to generate 100 channels of digital echo signals. It is preferable that the echo signal processor 32 performs the quadrature detection to the obtained analog echo signal while compensating for attenuation in accordance with the depth of tissue from which the echo waves are reflected and for frequency-dependent attenuation.

The first scan line data generator 26 has M first beamformers 33, for example, 24 first beamformers 33. The first beamformer 33 performs first reception focusing processes. In the first reception focusing processes, the first beamformer 33 reads all echo signals obtained by one transmission of ultrasonic beams or waves from the memory 24, and performs delay-and-sum of the read echo signals. Thereby, the first beamformer 33 generates the first scan line data or delay-and-sum RF data of one scan line. Using the 24 first beamformers 33, the first scan line data generator 26 generates 24 scan lines of the first scan line data per transmission of ultrasonic beams, in other words, first scan line data generator 26 focuses the echo signals received by each channel of the array transducer 21 to generate 24 scan lines of the first scan line data after a transmission of the ultrasonic beams. The 24 scan lines of the first scan line data are then inputted to the parallel/serial (P/S) converter 27.

The parallel/serial (hereinafter, abbreviated as P/S) converter 27 converts the first scan line data inputted from the first scan line data generator 26 into serial data, and then inputs the serial data to the wireless communication section 28. The P/S converter 27 serializes 24 scan lines of the first scan line data, generated per transmission of ultrasonic beams, as one unit. The wireless communication section 28 transfers the serialized first scan line data to the processing device 12 via an antenna 34. Additionally, when the wireless communication section 28 receives a control signal from the processing device 12 via the antenna 34, the wireless communication section 28 inputs the control signal to the controller 29. The transmission of the first scan line data is performed every time the transmission of the ultrasonic beams takes place.

The controller 29 controls overall operations of the ultrasonic probe 11. For example, the controller 29 inputs a control signal to the transmitter 22 to change the number (K) of ultrasonic transducers 31 to be driven per transmission of ultrasonic beams, a delay pattern of the ultrasonic transducers 31, and a shift amount of the transmission position (or group to be driven) of the ultrasonic transducers 31. Based on the changes, the transmitter 22 adjusts or changes the width or size of the ultrasonic beams (the size of the focal area or region) to which the ultrasonic beams converge, a focal depth, an overlapping portion or range of two successive transmissions, and the like. The controller 29 inputs a control signal to the receiver 23 to change the number (L) of ultrasonic transducers 31 which obtains echo signals of one transmission of ultrasonic waves. The controller 29 inputs a control signal to the first scan line data generator 26 to change the number (M) of the first scan line data generated per transmission of ultrasonic beams. The controller 29 controls each section of the ultrasonic probe 11 based on a control signal inputted from the processing device 12 via the wireless communication section 28 and a control signal inputted from an operation section 36 composed of a switch and the like. Power is supplied to each section of the ultrasonic probe 11 from a battery (not shown).

As shown in Fig. 2, the processing device 12 is composed of a wireless communication section 41, a serial/parallel (hereinafter abbreviated as S/P) converter 42, a memory 43, a second scan line data generator 44, an image generator 46, the monitor 47, a controller 48, and the like.

The wireless communication section 41 communicates with the ultrasonic probe 11 via an antenna 51 to receive the serialized first scan line data. The received first scan line data is inputted to the S/P converter 42. The wireless communication section 41 transmits a control signal inputted from the controller 48 to the ultrasonic probe 11 via the antenna 51. When the serialized first scan line data is inputted from the wireless communication section 41, the S/P converter 42 converts it back to parallel data, and then stores the parallel data in the memory 43. The memory 43 stores plural sets of first scan line data, and each set contains 24 scan lines of the first scan line data generated per transmission of the ultrasonic beams.

The second scan line data generator 44 is composed of N second beamformers 52, for example, 8 second beamformers 52. The second beamformer 52 reads from the memory 43 the plural pieces of first scan line data generated with the successive transmissions in different directions, for example, three transmissions of ultrasonic beams. The second beamformer 52 performs second reception focusing processes to the read first scan line data to generate the second scan line data of one scan line. In the second reception focusing processes, the second scan line is generated with the delay-and-sum process of the plural pieces of the first scan line data concerning the same scan line. In other words, the second scan line data generator 44 synthesizes second scan line data from the two or more first scan line data concerning a same scan line and obtained from transmissions in different directions. For example, the second beamformer 52 performs the second reception focusing processes to the first scan line data generated from echo of the (n-1)th transmission of the ultrasonic beams, the first scan line data generated from echo of the nth transmission, and the first scan line data generated from echo of the (n+1)th transmission. The second reception focusing processes are so-called aperture synthesis processing. In the second reception focusing processes, the delay-and-sum is not performed to the first scan line data generated with one transmission of ultrasonic beams. Instead, the delay-and-sum is performed to the plural pieces of the first scan line data generated with successive transmissions of the ultrasonic beams to generate or produce the second scan line data with improved resolution. The second scan line data has improved bearing resolution in the azimuth direction relative to the first scan line data. Because there are eight second beamformers 52, eight lines of the second scan line data are simultaneously generated. The generated second scan line data is input to the image generator 46.

The image generator 46 stores plural frames of second scan line data in a memory for image processing (not shown). To generate a cross-sectional image or ultrasonic tomographic image, the image generator 46 performs preprocesses to one frame of the second scan line data. The preprocesses include log compression and gain adjustment, and scan line conversion in which received data is converted into image data compliant to a normal scan method of TV signals. For example, the image generator 46 generates a cross-sectional image such as a B mode image and an M mode image in accordance with the setting in the processing device 12. The B mode represents a cross-section of a patient at a given time in a depth direction. The M mode image shows temporal changes in a single second scan line data. Thus, the cross-sectional image generated by the image generator46 is displayed on the monitor 47. A digital scan converter (DSC) is an example of the image generator 46.

The controller 48 controls overall operations of the processing device 12. An operation section 49 is composed of a keyboard, a pointing device, various buttons and dials. For example, when an operator operates the operation section 49 to input a control signal for changing the settings, the controller 48 inputs the control signal to each section of the processing device 12 to change the operation settings of each section. When a control signal to control the settings of the ultrasonic probe 11 is input via the operation section 49, the control signal is transmitted from the processing device 12 to the ultrasonic probe 11 via the wireless communication section 41 to allow the controller 29 of the ultrasonic probe 11 to control each section of the ultrasonic probe 11.

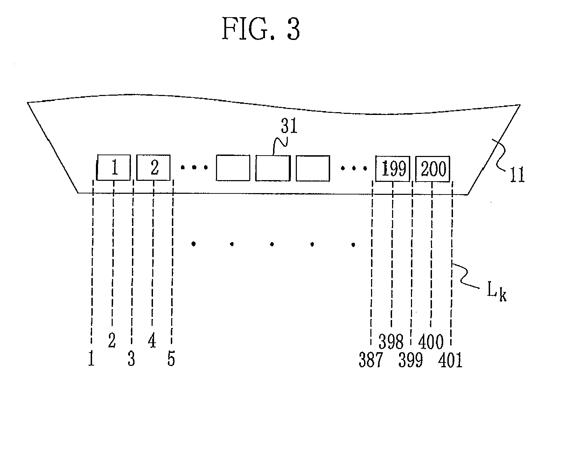

Next, an operation of the ultrasonic diagnostic apparatus 10 is described. Along the tip of the ultrasonic probe 11, from one end to the other end, 200 channels of the ultrasonic transducers 31 are arranged or aligned in a row. As shown in Fig. 3, for the ultrasonic diagnostic apparatus 10, two scan lines are obtained per ultrasonic transducer 31. To generate one (hereinafter referred to as one frame of) B mode image, the ultrasonic diagnostic apparatus 10 scans the patient using 401 scan lines Lk (k=1 to 401).

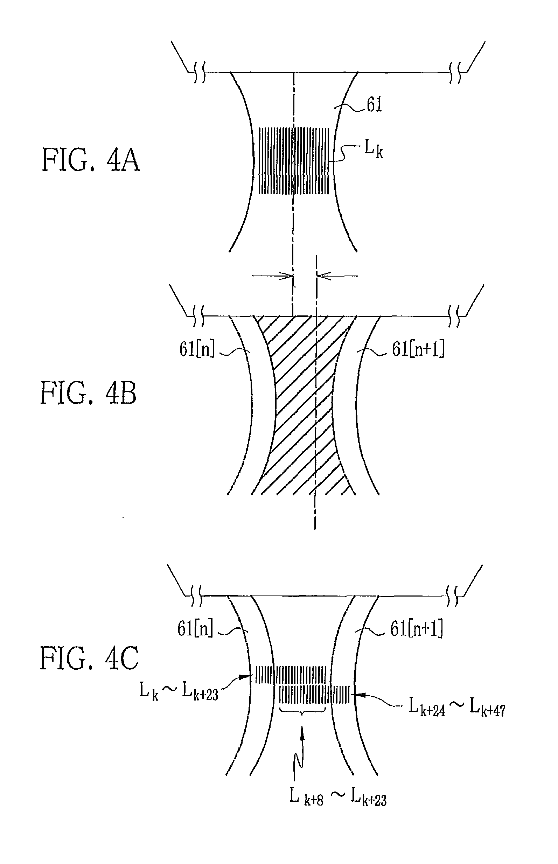

As shown in Fig. 4A, to observe the patient with the ultrasonic diagnostic apparatus 10, 12 adjacent ultrasonic transducers 31 are driven in a state that the ultrasonic probe 11 is contacted to the body of the patient. From the 12 adjacent ultrasonic transducers 31, wide ultrasonic beams 61 having 24 scan lines Lk (12 channels) are transmitted, which converge or focus to a wide area. The ultrasonic waves are reflected from each section inside the body of the patient, and returns to the ultrasonic probe 11 as echo waves. The receiver 23 selects 100 channels of the ultrasonic transducers 31 such that the ultrasonic transducers 31 which transmitted the ultrasonic waves are located at the center of the 100 channels excluding the ends, from among all the 200 channels to obtain 100 echo signals. Each of the obtained 100 echo signals are subjected to various signal processes to be converted into 100 channels of digital echo signals. Thereafter, in the ultrasonic probe 11, the first scan line data generator 26 performs the first reception focusing processes to the generated 100 channels of the digital echo signals to generate the first scan line data corresponding to each of 24 scan lines Lk in the ultrasonic beams 61. The generated 24 scan lines of the first scan line data are serialized every time the transmission of the ultrasonic beams 61 takes place, and transferred to the processing device 12 by radio.

As shown in Fig. 4B, when a transmission and reception of ultrasonic waves is ended, the ultrasonic probe 11 transmits the ultrasonic beams 61 while the center position of the group is shifted by four channels (eight lines) in a predetermined direction. Accordingly, the ultrasonic probe 11 transmits the ultrasonic beams 61 for 200/4 + 1 = 51 times to produce one frame of cross-sectional image or ultrasonic tomographic image. Because eight channels of ultrasonic transducers 31 of adjacent groups overlap with each other, the ultrasonic beams 61[n+1] transmitted in (n+1)th transmission and the ultrasonic beams 61[n]transmitted in nth transmission have an overlapping portion (a hatched portion in the drawing) (hereinafter, the number of transmission [n] is attached to the numeral 61 of the ultrasonic beams 61). As shown in Fig. 4C, the ultrasonic probe 11 generates the first scan line data Dk to Dk+23 corresponding to 24 scan lines Lk to Lk+23, respectively, based on the echo of the ultrasonic beams 61[n]. For the ultrasonic beams 61[n+1], the scan lines are shifted by eight scan lines (4 channels) in the shift direction of the ultrasonic beams 61, and the first scan line data Dk+24 to Dk+47 are generated corresponding to 24 scan lines Lk+24 to Lk+47, respectively. When the ultrasonic beams 61 [n] and the ultrasonic beams 61[n+1] are compared, the first scan line data Dk+8 to Dk+47 are generated with the 16 scan lines (8 channels) of the scan lines Lk+8 to Lk+23 overlapped. Thus, the ultrasonic probe 11 scans the object of interest while transmission ranges of the ultrasonic beams 61 are partly overlapped to generate 24 scan lines of the first scan line data after every transmission of the ultrasonic beams 61. The number of lines of ultrasonic beams is less than the number of the scan lines.

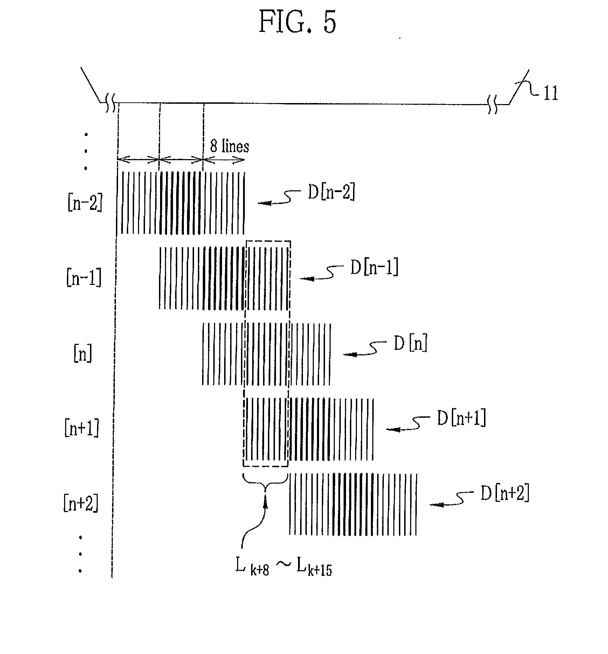

As shown in Fig. 5, the ultrasonic probe 11 generates three-fold or three pieces of the first scan line data for one scan line with the three successive transmissions of the ultrasonic beams. The first scan line data with the number three times as much as the number of the scan lines is transferred from the ultrasonic probe 11 to the processing device 12. For example, as shown by dashed lines, 8 scan lines of the first scan line data located at one end (the right end) of the first scan line data D[n-1] generated with (n-1)th transmission of the ultrasonic beams 61[n-1], 8 scan lines of the first scan line data located at the center of the first scan line data D[n] generated with nth transmission of the ultrasonic beams 61[n], and 8 scan lines of the first scan line data located at the other end (the left end) of the first scan line data D[n+1] generated with (n+1)th transmission of the ultrasonic beams 61[n+1] are generated for the same scan lines Lk+8 to Lk+15 with the different transmissions of the ultrasonic beams 61. Accordingly, in order to generate one frame of B mode image, the total number of the first scan line data generated by the ultrasonic probe 11 is three times as much as the number of the scan lines. In the case where an amount of data per scan line is defined as 1 unit (for example, approximately 80 KB), and the ultrasonic probe 11 transmits the ultrasonic beams 61 for 51 times per frame, an amount of data transferred from the ultrasonic probe 11 to the processing device 12 becomes 8 x 3 x 51 = 1224 units per frame.

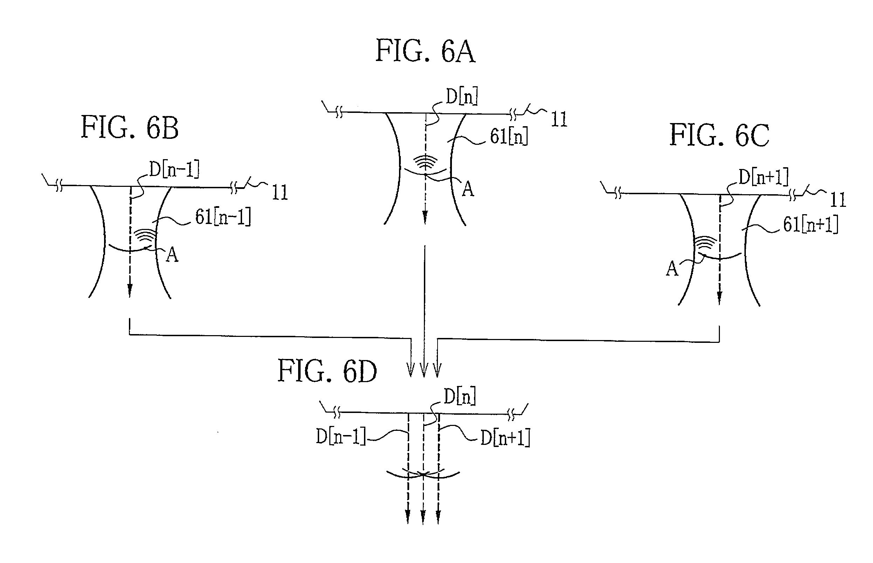

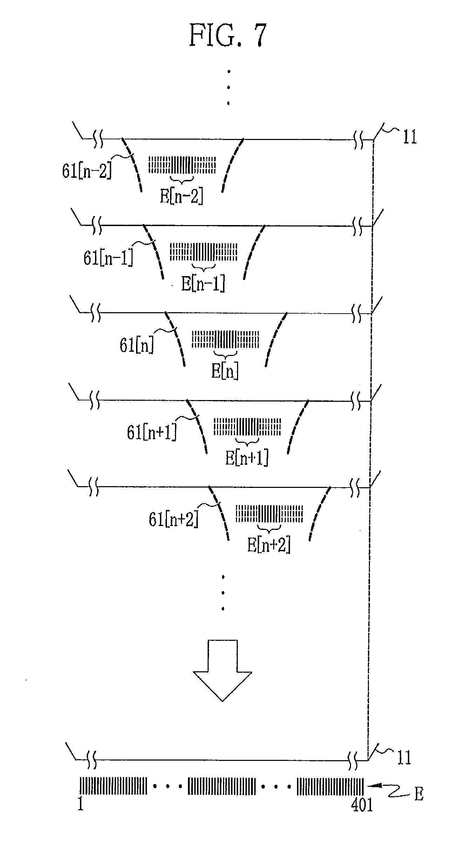

Thus, the ultrasonic probe 11 generates the first scan line data and transfers the generated first scan line data to the processing device 12. The processing device 12 stores the first scan line data in the memory 43. Thereafter, as shown in Figs. 6A to 6D, the second reception focusing processes are performed to the first scan line data to generate the second scan line data. In Fig. 6A, echo from tissue A located approximately at the center of ultrasonic beams 61[n] is shown as an example. As shown in Figs. 6B and 6C, a signal obtained from echo from the tissue A also appears in each of the first scan line data D[n-1] corresponding to the ultrasonic beams 61 [n-1] and the first scan line data D[n+1] corresponding to the ultrasonic beams 61[n+1]. As shown in Fig. 6D, the second scan line data generator 44 performs the delay-and-sum such that the positions of the signal of tissue A appeared in the first scan line data D[n-1] and D[n+1] match with the position of the signal of tissue A appeared in the first scan line data D[n]. By use of the first scan line data D[n-1], D[n], and D[n+1], the second scan line data generator 44 generates the second scan line data E[n](see Fig. 7) that corresponds to 8 scan lines located at the center of 24 scan lines of the first scan line data D [n]. The generated second scan line data E[n] has enhanced resolution in a direction in which the ultrasonic transducers 31 are aligned or arranged (azimuth direction) compared to the first scan line data D[n].

As described above, the second scan line data generator 44 performs delay-and-sum of the three-fold or three pieces of first scan line data generated or produced from the echo of three successive transmissions of ultrasonic beams to generate the second scan line data E[n]. The second scan line data E[n] corresponds to 8 scan lines located at the center of the first scan line data D[n] generated with the ultrasonic beams 61[n]. As shown in Fig. 7, the second scan line data generator 44 repeats the generation of the second scan line data E [n] for the number of transmission (51 times) of the ultrasonic beams 61. Thus, one frame of the second scan line data E is generated or produced. The image generator 46 generates a cross-sectional image based on the second scan line data E, and the generated cross-sectional image is displayed on the monitor 47.

As described above, in the ultrasonic diagnostic apparatus 10, the ultrasonic probe 11 does not transfer all the 100 channels of echo signals obtained after every transmission of the ultrasonic beams 61 to the processing device 12. Instead, the ultrasonic probe 11 performs first reception focusing processes to the obtained 100 channels of echo signals to generate 24 pieces (lines) of the first scan line data D. Accordingly, the amount of data transferred from the ultrasonic probe 11 to the processing device 12 becomes 1224 units per frame when an amount of data of one scan line is defined as one unit.

For a conventional line-by-line method which generates one scan line of scan line data per transmission of ultrasonic beams, in order to generate the same lines of scan line data as in the embodiment of the present invention, that is, 401 lines of scan line data from each of 100 channels of echo signals, the ultrasonic beams need to be transmitted 401 times per frame. With each transmission, 100 channels of the echo signal are transferred from the ultrasonic probe 11 to the processing device 12. For the typical line-by-line method, it is required to transfer data of 100 x 401 = 40100 units per frame.

For a simple zone sonography method, as with the ultrasonic diagnostic apparatus 10, the number of transmission of the ultrasonic beams 61 is reduced to 51 times, while 100 channels of echo signals are transferred from the ultrasonic probe 11 to the processing device 12 per transmission of the ultrasonic beams 61. In this case, the amount of data to be transferred from the ultrasonic probe 11 to the processing device 12 becomes 100 x 51 = 5100 units per frame.

In the present invention, the first scan line data D is generated in the ultrasonic probe 11, and then transferred to the processing device 12. Thereby, the amount of data transfer from the ultrasonic probe 11 to the processing device 12 is reduced to 1/16 compared to a typical line-by-line method, and to 1/4 compared to a simple zone sonography method. Reducing the amount of data transfer reduces power consumption of the data transfer, which enables the use of a thin cable with less number of wires. In the case where the wireless communication is used, power consumption of the ultrasonic probe 11 is reduced. This extends operation time of the ultrasonic probe 11, and thus the operability of the ultrasonic diagnostic apparatus 10 improves.

In the present invention, because the ultrasonic beams 61 are partly overlapped with each other, the three-fold or three pieces of scan line data are generated per one scan line with three successive transmissions of ultrasonic beams 61. The first scan line data is subjected to the second reception focusing processes to generate the second scan line data. The cross-sectional image is generated based on the second scan line data. Thereby, the ultrasonic diagnostic apparatus 10 produces the cross-sectional image having enhanced resolution in azimuth direction compared to the line-by-line method or zone sonography method, while an amount of data transfer from the ultrasonic probe 11 to the processing device 12 is reduced.

In the above embodiment, 200 channels of the ultrasonic transducers 31 are used, and 12 channels of ultrasonic beams 61 are transmitted, and 100 channels of the echo signals are received, as an example. It is preferable to change the number of channels to receive the echo signals depending on the properties and depth of the object of interest, a required frame rate, and the like, for example, via the operation section 49. With the change in the number of channels to receive the echo signals, the first scan line data in which resolution is adjusted in depth direction and in azimuth direction is generated. Generation of the first scan line data with the adjusted resolution makes the suitable frame rate for diagnosis compatible with the resolution of the cross-sectional image suitable for diagnosis.

In the above embodiment, 24 scan lines of the first scan line data are generated per transmission of the ultrasonic beams 61, as an example. It is preferable to change the number of scan lines for generating the first scan line data (the number of the first scan line data to be generated) in accordance with the width or the size (the size of a focal region) of the ultrasonic beams 61 and an overlapping portion of the ultrasonic beams 61, a communication status between the ultrasonic probe 11 and the processing device 12, and a frame rate required for the observation. As described above, in the ultrasonic diagnostic apparatus 10, the number of the scan lines used for generating the first scan line data per the transmission of the ultrasonic beams 61 can be changed through an input from the operation section 49 or the like. Additionally, with a change in the size (thickness) of the ultrasonic beams 61 and/or a pitch to shift the ultrasonic transducers 31 to be driven, a width (the size of the focal region) of the ultrasonic beams 61 of each of the successive transmissions can be changed. For example, when the width of the ultrasonic beams 61 is changed, it is preferable that the controller 29 changes the number of the first scan line data to be generated in the first scan line data generator 26 in accordance with the changed width of the ultrasonic beams 61. As with the above embodiment, to generate the three pieces of or three-fold first scan line data per scan line, the number of the first scan line data may be changed such that the first scan line data with the number as three times as much as the number of the scan lines within the overlapping portion is generated per transmission of the ultrasonic beams 61.

In the case where observation at a high frame rate is required because of, for example, poor communication between the ultrasonic probe 11 and the processing device 12, artifact, or the like, it is preferable to reduce the number of scan lines for generating the first scan line data per the transmission of the ultrasonic beams 61 to reduce the data transfer amount between the ultrasonic probe 11 and the processing device 12. Specifically, when the number of the scan lines is reduced based on the communication status between the ultrasonic probe 11 and the processing device 12, it is preferable that the ultrasonic probe 11 and the processing device 12 are provided with a circuit for monitoring the communication status and outputting a signal corresponding to the communication status. It is preferable to automatically change the number of ultrasonic transducers in one group to change the number of scan lines in accordance with the output signal. Changing the number of the first scan line data optimizes the amount of data processing in the ultrasonic probe 11 and the amount of data transfer to the processing device 12 in accordance with a kind or type of diagnosis.

In the above embodiment, the ultrasonic beams 61 are transmitted while being shifted by 4 channels (8 scan lines). The pitch (a shift amount) of the ultrasonic beams may be changed to any value as long as the scan lines for generating the first scan line data partly overlap with each other at the transmission of the ultrasonic beams 61. In the above embodiment, the controller 29 automatically adjusts the pitch to an appropriate value. In the case where the number of the scan lines is changed, it is preferable to change the pitch depending on the width of the ultrasonic beams 61 and the changed number of the scan lines, and it is more preferable to automatically change the pitch.

In the above embodiment, the first scan line data is transferred from the ultrasonic probe 11 to the processing device 12 via wireless transmission as an example. Alternatively, the ultrasonic probe 11 and the processing device 12 may be connected via a communication cable to transfer the first scan line data. As with the above embodiment, the echo signal itself is not transferred via the cable. The first scan line data is generated in the ultrasonic probe 11, and the first scan line data is transferred to the processing device 12 to reduce the amount of data transfer between the ultrasonic probe 11 and the processing device 12. As a result, the number of wires is reduced, which reduces the diameter of the communication cable. Thus, operability of the ultrasonic diagnostic apparatus 10 is improved.

In the above embodiment, the first scan line data generated with three successive transmissions of the ultrasonic beams 61 is subjected to the second reception focusing processes to generate the second scan line data, as an example. The second scan line data may be generated from the first scan line data of two, or four or more successive transmissions of the ultrasonic beams 61. At least two pieces of the first scan line data is necessary to generate the second scan line data. It is especially preferable to generate the second scan line data from the first scan line data of three successive transmissions because of a good balance between generation speed of the second scan line data and resolution of the cross-sectional image.

In the above embodiment, the first scan line data generated with the successive transmissions of ultrasonic beams is subjected to the second reception focusing processes to generate or produce the second scan line data with improved bearing resolution. Alternatively or in addition, contrast resolution of the second scan line data may be improved compared to the first scan line data using the second reception focusing processes, or both the bearing resolution and the contrast resolution may be improved using the second reception focusing processes.

In the above embodiment, the first beamformer 33 generates the first scan line data per scan line, and the first scan line data generator 26 is composed of the first beamformers 33 with the same number as the number of the first scan line data Dk(in the above embodiment, "24"), for example. Alternatively, for example, the first scan line data generator 26 may be composed of one first beamformer 33, and this first beamformer 33 may sequentially generate 24 scan lines of the first scan line data Dk. In this case, 24 scan lines of the first scan line data Dk may be generated using time-division processing. Alternatively, the first scan line data generator 26 may be composed of plural first beamformers 33 with the number less than 24, and the first beamformers 33 may share the generation of 24 scan lines of the first scan line data Dk. These descriptions also apply to the second scan line data generator 44 and the second beamformers 52.

In the above embodiment, the ultrasonic beams 61 are scanned while the group of the ultrasonic transducers 31 to be driven is shifted to transmit the ultrasonic beams 61 in different directions, for example. Alternatively, for example, the present invention may be applied to a so-called sector type ultrasonic probe that changes a transmission direction of ultrasonic beams 61 using all the ultrasonic transducers 31 in the array transducer 21. In the above embodiment, the center position of the ultrasonic beams 61 is shifted, for example. The shifting of the center position may include adjustment of transmission angle of the ultrasonic beams 61 depending on a type or configuration of the ultrasonic probe.

In the above embodiment, the first scan line data is generated from the echo signals which have been subjected to quadrature detection in the ultrasonic probe 11, as an example. The quadrature detection may be performed at any timing within the ultrasonic probe 11. For example, the echo signal outputted from the ultrasonic transducers 31 are digitized, and then subjected to the first reception focusing processes to generate the first scan line data. Thereafter, the first scan line data is subjected to the quadrature detection, and then transferred to the processing device 12. In the above embodiment, the echo signal as a complex baseband signal is used in data processes after the first scan line data is generated, for example. The echo signal may be converted into the complex baseband signal at any timing.

In the above embodiment, B mode images and M mode images are described as examples of cross-sectional images or ultrasonic tomographic images generated based on the second scan line data. Other well-known cross-sectional images such as Doppler mode images and images in which elasticity index and the like are visualized may be generated based on the second scan line data.

Various changes and modifications are possible in the present invention and may be understood to be within the present invention.

## Claims

1. An ultrasonic diagnostic apparatus (10) including an ultrasonic probe (11) and a processing device (12), comprising:
A. the ultrasonic probe including:
an array transducer (21) having plural ultrasonic transducers (31) for transmitting ultrasonic beams to an inside of a subject and receiving echo waves to output echo signals;
a first scan line data generator (26) for focusing the echo signals received by each channel of the array transducer and generating two or more first scan line data after a transmission of the ultrasonic beams, a data set of the first scan line data being sent to the processing device;
B. the processing device including:
a second scan line data generator (44) for synthesizing second scan line data from the two or more first scan line data concerning a same scan line and obtained from the transmissions in different directions; and
an image generator (46) for generating an ultrasonic image of the subject based on the second scan line data.

2. The ultrasonic diagnostic apparatus of claim 1, wherein the number of lines of ultrasonic beams is less than the number of the scan lines, and a wide beam is transmitted such that the adjacent ultrasonic beams are partly overlapped.

3. The ultrasonic diagnostic apparatus of claim 2, wherein the first scan line data generator generates the two or more first scan line data concerning the same scan line using the echo signals obtained with successive ultrasonic beams, and the second scan line data is generated from the two or more first scan line data concerning the same scan line.

4. The ultrasonic diagnostic apparatus of claim 3, wherein the ultrasonic probe further includes a controller (29) for controlling a width to which the ultrasonic beams converge, and the first scan line data generator changes the number of the first scan line data depending on the width to which the ultrasonic beams converge.

5. The ultrasonic diagnostic apparatus of claim 4, wherein the controller changes a shift amount of the ultrasonic beams in accordance with the width to which the ultrasonic beams converge.

6. The ultrasonic diagnostic apparatus of claim 3, wherein the ultrasonic probe further includes a conversion section (32) for performing quadrature detection to the echo signal to convert the echo signal into a complex baseband signal, and the first scan line data generator and the second scan line data generator generate the first scan line data and the second scan line data, respectively, based on the complex baseband signal.

7. The ultrasonic diagnostic apparatus of claim 3, wherein the first scan line data is sent from the ultrasonic probe to the processing device via wireless communication.

8. A method for operating an ultrasonic diagnostic apparatus (10), the ultrasonic diagnostic apparatus having an ultrasonic probe (11) and a processing device (12), the method comprising the steps of:
driving plural ultrasonic transducers (31) of an array transducer (21) provided in the ultrasonic probe and transmitting ultrasonic beams to an inside of a subject;
receiving echo waves with the plural ultrasonic transducers and outputting echo signals from the ultrasonic transducers after a transmission of the ultrasonic beams;
focusing the echo signals received by each channel of the array transducer and generating two or more first scan line data in a first scan line data generator (26) in the ultrasonic probe;
sending a data set of the first scan line data from the ultrasonic probe to the processing device;
synthesizing second scan line data in a second scan line data generator (44) in the processing device, the second scan line data being synthesized from the two or more first scan line data concerning a same scan line and obtained from the transmissions in different directions; and
generating an ultrasonic image of the subject based on the second scan line data in an image generator (46) in the processing device.

9. The method of claim 8, wherein the number of lines of ultrasonic beams is less than the number of the scan lines, and a wide beam is transmitted such that the adjacent ultrasonic beams are partly overlapped.
